# EUROPÄISCHE PATENTANMELDUNG

(11) **EP 3 753 536 A1**
(43) Veröffentlichungstag der Anmeldung: **23.12.2020**
(21) Anmeldenummer: 20174123.8
(22) Anmeldetag: 12.05.2020
(51) Int. Cl.: A61F 2/58, A61F 2/50, A61F 2/68

(54) **RÜCKMELDUNGSSYSTEM FÜR EINE PROTHESE, INSBESONDERE FÜR EINE ARMPROTHESE, UND VERFAHREN ZUM ERZEUGEN EINES STIMULUS FÜR DEN TRÄGER DER PROTHESE MITTELS EINES SOLCHEN RÜCKMELDUNGSSYSTEMS**

(30) Priorität: 18.06.2019 AT 505482019
(71) Anmelder: Kos, Tomaz, 4201 Zgornja Besnica (SI)
(72) Erfinder: Kos, Tomaz, 4201 Zgornja Besnica (SI)
(74) Vertreter: Flügel Preissner Schober Seidel

(57) **Zusammenfassung**

Ein Rückmeldungssystem (10) für eine Prothese eines Patienten (100) umfasst einen Handschuh (11); mindestens einen Sensor (12a, 12b, 12c), der ein Messsignal (25) erzeugt; wenigstens zwei einander ergänzende Rückmeldungseinheiten (13, 14, 15), die kontinuierlich jeweils wenigstens einen Feedback-Stimulus (28) erzeugen; eine Verarbeitungseinheit (16), die das Messsignal (25) in ein Feedback-Signal (26a, 26b) an die Rückmeldungseinheiten (13, 14, 15) umwandelt; und eine Speichereinheit (17), die das Messsignal (25) und zudem vorgegebene Grenzwerte (21, 23), die wenigstens drei Belastungsbereiche (20, 22, 24) voneinander abgrenzen, speichert. Der dritte Belastungsbereich (24) liegt oberhalb eines zweiten Grenzwerts (23), der zweite Belastungsbereich (22) liegt zwischen einem ersten Grenzwert (21) und dem zweiten Grenzwert (23) und der erste Belastungsbereich (20) liegt unterhalb des ersten Grenzwerts (21). Die Rückmeldungseinheiten (13, 14, 15) sind derart ausgestaltet, dass aus dem Feedback-Signal (26a, 26b) wenigstens ein Feedback-Stimulus (28) erzeugt wird, der den Belastungsbereich (20, 22, 24) für den Patienten (100) angibt.

## Beschreibung

Die Erfindung betrifft ein Rückmeldungssystem für eine Prothese, insbesondere für eine Armprothese, und ein Verfahren zum Erzeugen eines Stimulus für den Träger der Prothese, im Folgenden auch Patienten genannt, mittels eines solchen Rückmeldungssystems.

Eine Prothese im Sinne der vorliegende Erfindung umfasst sowohl myoelektrisch gesteuerte Prothesen, die auf die Muskelsignale ihres Trägers reagieren, als auch passive Prothesen, sogenannte Habitus-Prothesen, bei denen das optische Erscheinungsbild im Vordergrund steht und die sich passiv verhalten, also lediglich ein Gegenhalten bei der Handhabung eines Gebrauchsgegenstandes ermöglichen. Eine Armprothese im Sinne der vorliegenden Erfindung kann eine Hand- und Teilhandprothese, eine Fingerprothese, eine Unterarmprothese oder eine Oberarmprothese sein.

Weltweit gibt es mehr als 3 Millionen Menschen, welchen eine obere Körperextremität fehlt. In den USA alleine kommen jährlich 10 000 neue Patienten aufgrund einer Krankheit, einem angeborenen Defekt oder einem Unfall hinzu. Der Verlust eines Körperglieds resultiert in einer ernstzunehmenden psychologischen Beeinträchtigung, die zu Depressionen, aber auch zu chronischen, medizinischen Problemen, wie beispielsweise Phantomschmerzen, führen kann [A. Vázguez PI, et al. (2018): Prevalence and correlations between suicide attempt, depression, substance use, and functionality among patients with limb amputations*;* M. Carolien, et al. (2000): Phantom pain and phantom sensations in upper limb amputees: an epidemiological study]. Dadurch kann des Weiteren die Leistungsfähigkeit reduziert oder das Arbeitsleben beeinträchtigt werden. Es gibt hierfür jedoch auch bewährte Methoden, die es dem Patienten ermöglichen, mittels fortschrittlichen Prothesen die Rehabilitation oder die Integration in der Gesellschaft zu unterstützen. Gemäß verschiedener Studien hat sich jedoch gezeigt, dass das Fehlen eines sensorischen oder haptischen Feedbacks eines der größten Nachteile solcher Prothesen ist [B. Peerdeman, et al. (2011): Myoelectric forearm prostheses: State ofthe art from a user-centered perspective*;* F. Cordella, et al. (2016) Literature Review on Needs of Upper Limb Prosthesis Users].

Sensorische Feedback-Systeme, die dem Patienten nach dem Verlust eines Arms oder Beins mittels eines künstlichen, sensorischen Feedback-Signals unterstützen, sind seit den 70er Jahren bekannt [R. E. Prior, et al. (1976): Supplemental sensory feedback for the VA/NU myoelectric hand: background and feasibility*;* R. R. Riso (1999) Strategies for providing upper extremity amputees with tactile and hand position feedback--moving closerto the bionic arm]. Jedoch wurde die Bedeutung einer sensorischen Feedback-Funktion als wesentlicher Bestandteil der Prothese bisher unterschätzt. Derartige Funktionen kommerziell zu nutzen, war jedoch bisher aufgrund der begrenzten, technischen Möglichkeiten bis 2016 nicht umsetzbar. [S. Schulz, et al. (2014) New Strategies for Myoelectric Control of Multi-Articulating Hand and Partial Hand Prostheses]. Sensorisches Feedback ist ein direkter Bestandteil eines kontrollierten Bewegungsablaufs [B. Peerdeman, et al. (2011): Myoelectric forearm prostheses: State of the art from a user-centered perspective]. Der jeweilige Stimulus (visuell, haptisch, akustisch, etc.), den das Gehirn in einem spezifischem Moment verarbeitet, ist von der vorherrschenden Situation abhängig. In diesem Sinne gibt es nicht einen idealen Übertragungsstimulus, vielmehr wählt das Gehirn situationsabhängig den für sich geeigneten Stimulus aus. Hierfür wird generell bei bestehenden Systemen jeweils nur ein Stimulus mittels einer künstlichen, sensorischen Feedback-Vorrichtung stimuliert.

Ein System, das ein bewusstes sensorisches Feedback einer empfindungslosen Körperextremität oder einer fehlenden Körperextremität eines Patienten ermöglicht, ist aus EP 1 861 049 B1 bekannt. Das System weist einen Sensor, eine taktile Anzeige und einen Messwertgeber, der an der Haut einer intakten, angrenzenden Körperextremität des Patienten angeordnet wird, auf. Dabei besitzt mindestens ein Sensor die Fähigkeit, wenn er mindestens einem Stimulus ausgesetzt wird, das Signal in den Stimulus auf natürlich auftretende Nervenkomponenten in der Haut der benachbarten intakten Körperextremität umzusetzen. Die taktile Anzeige weist eine Halterung auf, auf der die Messwertgeber in einem räumlichen Muster angeordnet sind, das einem räumlichen Muster der Sensoren entspricht, die an der empfindungslosen Körperextremität oder der Körperextremitätsprothese angeordnet sind. Jedoch muss der Patient die taktilen Signale, die er über die Messwertgeber an der Haut einer intakten Körperextremität übertragen bekommt, unterscheiden können. Räumliche Unterscheidung und die Wahrnehmung der Größe einer Kraft mittels einer kontinuierlichen Änderung eines Signals bedarf eines vorhergehenden Anlernprozesses und gleichzeitig die volle Aufmerksamkeit des Patienten zur Interpretation des Signals.

Weiterhin offenbart US 2017/0119553 A1 eine haptische Feedback-Vorrichtung, die auf der Prothese oder einer empfindungslosen Körperextremität eines Patienten angebracht ist. Die Vorrichtung umfasst Sensoren, eine taktile Stimulationseinheit und einen Handschuh als eine Tragevorrichtung, sowie eine Kontrolleinheit, welche zyklisch das Kontaktsignal mit einem Grenzwert vergleicht. Dem Patienten einer solchen Tragevorrichtung steht jedoch nur die taktile Stimulation relativ zu einer Änderung der auf die Prothese wirkenden Kraft, die über die Tragevorrichtung auf den Patienten übertragen wird, zur Verfügung. Eine detaillierte Rückkopplung über die gemessene Greifkraft, steht dem Patienten jedoch nicht zur Verfügung.

US 2014/0277588 A1 offenbart ein System, wodurch eine Prothese mit einem nicht taktilen Sensor-Feedback versehen ist. Das beschriebene System umfasst ein Leuchtelement, welches visuelle Hinweise auf eine Berührung aufzeigt, einen kleinen elektronischen Bildschirm oder einen anderen nicht taktilen Feedback-Mechanismus, welcher an einer Armprothese oder einem anderen Körperglied angebracht ist. Eine kontinuierliche Änderung der Farbnuancen benötigt sowohl die volle Aufmerksamkeit des Patienten als auch einen vorhergehenden Anlernprozess.

Eine Vorrichtung zum Rückkoppeln der Greifkraft einer Greifvorrichtung an den Bediener ist aus der DE 10 2010 007 419 A1 bekannt. Hierbei erfolgt die Rückkopplung der erfassten Greifkraft an den Bediener, indem über einen Aktor eine von der Greifkraft abhängige Rückkopplungskraft auf den Arm des Bedieners aufgebracht wird. Die mechanische Anbindung ermöglicht keine detaillierte und filigrane Differenzierung von kritischen Momenten, wie zum Beispiel bei Berührungen oder dem Halten eines Gegenstands, oder der dynamischen Anpassung an die Situation des Patienten. Dem Patienten steht somit nur die applizierte Kraft zur Interpretation der Greifkraft zur Verfügung.

WO 2019/025 838 A1 offenbart eine Beinprothesen-Sensor-Stimulator Anordnung mit einem Sensorteil für eine Beinprothesensohle. Die Beinprothesen- Sensor- Stimulator Anordnung umfasst einen Sensorteil für die Beinprothesensohle, die in gängigen Sohlenabschnitten der Sohle über einen Sensor verfügt, der über eine Datenverbindung und wenigstens eine Steuerung mit einem nervenanregenden Stimulator zur Informationsübertragung an einen physionomietypischen Nervenarealabschnitt verbunden ist. Besonders gute Ergebnisse werden nach einem chirurgischen Eingriff erwartet, wobei gezielt eine Reinnervation durchgeführt wird.

Eine derartige Vorrichtung ist jedoch für die auftretenden Belastungsarten bei einer Beinprothese vorgesehen. Dabei wird über mehrere Sensoren eine Unterstützung der Laufbewegung des Patienten über eine kontinuierliche Änderung der Feedback-Signale dem Patienten zur Verfügung gestellt, wobei jedoch nur ein Stimulus angeregt wird.

Die bekannten Lösungen beruhen auf einer einfachen Rückkopplungslogik. Die Vielzahl an verschiedenen Belastungsarten und Belastungsaufgaben, die im Alltag eines Patienten auftreten, werden indes nicht berücksichtigt. Es ist jedoch für den Patienten wichtig, ein detailliertes, intuitives und zugleich leicht interpretierbares Feedback-Signal zur Verfügung zu haben, sodass sich die Handhabung einer Prothese fließend in den Alltag des Patienten integrieren lässt.

Der Erfindung liegt die Aufgabe zugrunde, ein Rückmeldungssystem vorzuschlagen, das einfach und für den Patienten leicht zu interpretieren ist. Des Weiteren wird ein Verfahren vorgeschlagen, mittels welchem eine für den Patienten leicht verständliche Rückkopplung vermittelt wird. Insbesondere soll dem Patienten ein flexibles System zur Verfügung gestellt werden, das unterschiedliche, sensorische Feedback-Stimuli bereitstellt, sodass der Patient für sich seinen optimalen, der Situation angepassten Feedback-Stimulus auswählen kann.

Diese Aufgabe wird durch ein Rückmeldungssystem gemäß Anspruch 1 und einem Verfahren gemäß Anspruch 13 gelöst. Bevorzugte Ausgestaltungen der Erfindung sind Gegenstand der Ansprüche 2 bis 12 sowie 14 und 15.

Das erfindungsgemäße Rückmeldungssystem umfasst einen Handschuh, der die Prothese des Patienten zumindest teilweise umschließt, und mindestens einen Sensor, der an dem Handschuh angeordnet ist und ein Messsignal erzeugt. Das Rückmeldungssystem umfasst ferner wenigstens zwei einander ergänzende Rückmeldungseinheiten, die kontinuierlich einen Feedback-Stimulus erzeugen, durch das mindestens ein Sinnesorgan des Patienten mittels eines akustischen, vibrierenden, optischen und/oder elektrischen Stimulus stimuliert wird. Weiterhin umfasst das Rückmeldungssystem eine Verarbeitungseinheit, die das Messsignal in einen Befehl, das sogenannte Feedback-Signal, an die Rückmeldungseinheiten umwandelt, und eine Speichereinheit, die das Messsignal und zudem vorgegebene Grenzwerte, die wenigstens drei Belastungsbereiche voneinander abgrenzen, speichert. Aufgrund der mindestens zwei vorhandenen Rückmeldungseinheiten wird dem Patienten unabhängig von seiner durch die Umgebung in Anspruch genommener Aufmerksamkeit ein Feedback-Stimulus vermittelt, welchen er erkennt. Der besondere Vorteil ergibt sich dadurch, dass der Patient einen zusätzlichen Feedback-Stimulus vermittelt bekommt und bei Bedarf seine Aufmerksamkeit beiden Signalen widmen kann, wodurch ihm die Kontrolle der Prothese erleichtert wird. Die verschiedenen Belastungsbereiche, die ein deutlich voneinander zu unterscheidendes Signal aufweisen, ermöglichen es dem Patienten, in einer intuitiven und verständlichen Art wahrzunehmen, welche Messgröße an den Sensoren anliegt. In einer vorteilhaften Ausgestaltung kann der Handschuh auch als Schuh für Bein- oder Fußprothesen ausgebildet sein.

Mittels der Kombination mehrerer Feedback-Stimuli können die Nachteile einzelner Feedback-Stimuli ausgeglichen werden. Beispielhaft kann über die Haut nur bedingt zwischen verschiedenen taktilen Mustern unterschieden werden [K. A. Kaczmarek, et al. (1991) A tactile vision-substitution system for the blind: computer-controlled partial image sequencing]. Dies bedeutet auch, dass es dem Patienten eine hohe Konzentration abverlangt, ein kontinuierliches Feedback-Stimulus zu interpretieren. Visuelle und/oder akustische Stimuli können mit einer hohen Auflösung wahrgenommen werden, sodass verschiedene Muster oder Änderungen sehr leicht und eindeutig interpretiert werden können. Diese Feedback-Stimuli sind bei einer präzisen Anwendung der Prothese vorteilhaft, die eine exakte Kontrolle der Bewegung bei vollem Bewusstsein des Patienten für eine bestimmte Zeit beansprucht.

Die verschiedenen besonders einfach zu interpretierenden Feedback-Stimuli ermöglichen somit eine intuitive Benutzung der Prothese, die schnell von dem Patienten angenommen werden können. Dabei werden besonders durch die unterschiedlichen, zur Verfügung stehenden Stimuli, akustisch, visuell und taktil, eine intuitive und leicht verständliche Rückkopplung der Bewegung ermöglicht. Das erfindungsgemäße System berücksichtigt darüber hinaus die limitierte Fähigkeit der Haut Unterschiede des taktilen Feedback-Stimulus wahrzunehmen. In vorteilhafter Weise kann somit eine myoelektrische Hand- oder Armprothese dahingehend verbessert werden, dass das Greifen von Gegenständen ermöglicht, das Entgleiten von Gegenständen aus der Hand verhindert, ein zu festes Greifen von Gegenständen bewusst oder sogar ein Handschlag kontrolliert durchgeführt werden kann.

In einer bevorzugten Ausgestaltung der Erfindung ist das Feedback-Signal unstetig, das heißt, dass das Feedback-Signal sich sprunghaft verändern kann. Daraus ergibt sich ein Feedback-Stimulus, welcher sich beispielsweise in seinem Rhythmus, Intensität, Lautstärke oder Farbnuancen vollkommen ändert.

Wenngleich sich das erfindungsgemäße Rückmeldungssystem dadurch auszeichnet, dass es in der Lage ist, ein sehr geringes Messsignal, das sich beispielsweise schon bei einer leichten Berührung oder einem Nahezukontakt ergibt, in einen Stimulus umzuwandeln, kann es bei Bedarf zweckmäßig sein, einen Schwellenwert vorzusehen, oberhalb dessen der ersten Belastungsbereich anfängt. Dadurch ergibt sich der Vorteil, dass in einer Ruhephase dem Patienten kein Feedback-Signal über die Rückmeldungseinheiten übermittelt wird. Ebenfalls kann ein sogenanntes Grundrauschen in dem Signal ausgeschlossen werden.

Vorteilhaft ist der Handschuh aus einem ersten Material, insbesondere Silikon oder Polyvinylchlorid (PVC), das einen ersten Elastizitätsmodul hat, und wenigstens einem zweiten Material, insbesondere Textil oder Faser, das einen zweiten Elastizitätsmodul hat, der größer ist als der erste Elastizitätsmodul. Vorzugsweise sind das erste Material und das zweite Material übereinander und/oder nebeneinander angeordnet. Aufgrund der Flexibilität der beiden Materialen können diese leicht über die Prothese gestülpt werden. Ebenfalls kann das Rückmeldungssystem aufgrund einer solchen Ausgestaltung für unterschiedlich ausgeprägte Prothesen verwendet werden. Das erste Material kann flüssigkeitsabweisend sein und bietet der Prothese zusätzlich Schutz gegenüber Verschmutzungen, da das erste Material in diesem Fall eine geschlossene Oberfläche über sensible Abschnitte der Prothese aufweisen kann. Das zweite Material prägt die Passung, wodurch insbesondere die Position der Sensoren fixiert wird. Aufgrund der flexiblen Ausgestaltung der für den Handschuh verwendeten Materialien ist die Bewegungsfreiheit der Prothese nicht beeinträchtigt.

In vorteilhafter Weise ist der Sensor ausgestaltet, Druck, Kapazität, Temperatur, Spannung oder eine sonstige Größe, die geeignet ist, einen wahrnehmbaren Kontakt und/oder Nahezukontakt zu erfassen, zu messen, wodurch es dem Patient ermöglicht wird, verschiedene, situationsbedingte Bedingungen zu erfassen, die er sich jeweils über die Rückmeldungseinheiten anzeigen lassen kann.

In einer vorteilhaften Ausgestaltung ist der Sensor im Bereich des dritten Fingergliedes der Finger der Prothese, insbesondere im Bereich des dritten Fingergliedes des Daumens, angeordnet. Aufgrund einer solchen Verteilung kann eine detaillierte Information über die Bewegung eines Fingers, insbesondere bei einem Greifvorgang, ermittelt werden.

Ferner ist es vorteilhaft, wenn der Sensor in den Handschuh integriert ist. Der Sensor ist somit von dem ersten Material des Handschuhs geschützt umfasst. Entsprechend sind die Sensoren somit nicht der Umgebung und folglich beispielsweise der Feuchtigkeit ausgesetzt. Ebenfalls sind die Sensoren somit gegenüber Abnutzungen durch Reibung geschützt.

In einer vorteilhaften Ausprägung des Handschuhs, welcher eine obere Seite, die den Handrücken abdeckt, und eine untere Seite, welche die Handfläche abdeckt, aufweist, befindet sich vorzugsweise der Sensor an der unteren Seite des Handschuhs. Somit lässt sich die Messgröße nachvollziehen, die über den gesamten Bereich der Handfläche während eines Händedrucks erfasst wird. Ferner kann eine räumlich-geometrische Darstellung der Druckstellen auf der Handfläche angezeigt werden.

Vorteilhaft sind auf dem Handschuh eine Vielzahl an Sensoren angeordnet, die vorzugsweise als Array angeordnet und weiter vorteilhaft auf dem Handschuh verteilt angeordnet sind. Eine solche Verteilung weist den besonders vorteilhaften Effekt auf, dass Berührungen mit den verschiedenen Bereichen des Handschuhs gemessen werden können. Eine umfassende, detaillierte Erfassung über alle Flächen der Hand kann somit erfolgen, wodurch es dem Patienten möglich ist, ein detailliertes Abbild der Messwertgrößen der einzelnen Bereiche zu bekommen.

Vorteilhaft weist der Handschuh einen die Finger umfassenden distalen Bereich und einen proximalen Bereich auf, wobei vorzugsweise die Verarbeitungseinheit im proximalen Bereich des Handschuhs angeordnet ist. Unter proximalen Bereich ist der Bereich des Handschuhs zu verstehen, der dem Körper des Patienten zugewandt ist, wohingegen der distale Bereich, als ein dem Körper des Patienten abgewandter Bereich zu verstehen ist. Als Finger einer Prothese ist der Bereich der Prothese zu verstehen, der vergleichbar in der Nutzung zu den Fingern einer menschlichen Hand gesehen werden kann. Aufgrund dieser Anordnung der Sensoren und Verarbeitungseinheiten wird zum einen das Messsignal nicht gestört und zum anderen ist die Gewichtsverteilung verbessert, da das Gewicht der Verarbeitungseinheit nahe am Körper getragen werden kann und sich somit das Moment verringert, welches durch die Distanz von einem Gelenk des Arms und dem Gewicht, insbesondere dem Schwerpunkt, der Verarbeitungseinheit gebildet wird. Aufgrund dieser verkürzten Distanz ist ferner das Trägheitsmoment des Systems geringer und eine dynamischere Handhabung ergibt sich in Folge dessen.

Die Rückmeldungseinheit und die Verarbeitungseinheit können mittels eines Kabels miteinander verbunden sein. Es hat sich jedoch als besonders vorteilhaft erwiesen, die Rückmeldungseinheit und die Verarbeitungseinheit mittels eines kabellosen Datenübertragungsmediums, beispielsweise nach dem Bluetooth-Standard oder dem IEEE-802.11-Standard, miteinander zu verbinden. Somit kann die Rückmeldungseinheit beliebig in dem von dem Patienten bevorzugten Bereich getragen werden.

In einer weiteren Ausgestaltung ist die Rückmeldungseinheit als Leuchtelement, vorzugsweise LED, als Vibrationsmotor, als Druckaktuator oder als Lautsprecher ausgebildet. Ferner ist es denkbar, dass das Leuchtelement ein Display umfasst, welches die genaue Position und die Messwerte der einzelnen Sensoren anzeigt. Diese Anzeige kann die räumliche und geometrische Anordnung der Sensoren des Handschuhs anzeigen. Neben dem Unterschied in der Anzeige der Farbe oder eines Musters an verschiedenen Farben kann sich die Lichtintensität des Leuchtelements in Abstufungen von zweckmäßigerweise 20 Lumen vollkommen ändern. Der Vibrationsmotor ist als Motor zu verstehen, der einen taktilen Stimulus erzeugen kann. Dieser taktile Feedback-Stimulus weist eine Frequenz zwischen 10 Hz und 500 Hz, zweckmäßigerweise zwischen 15 Hz und 250 Hz, am bevorzugtesten jedoch zwischen 190 Hz und 210 Hz auf , da diese Frequenz sehr gut von dem menschlichen Tastzellen erkannt wird.

Des Weiteren wird zur Lösung der Aufgabe ein Verfahren zum Erzeugen eines Stimulus für einen Patienten mittels des erfindungsgemäßen Rückmeldungssystems vorgeschlagen, bei dem aus dem wenigstens einen Feedback-Signal durch die wenigstens zwei Rückmeldungseinheiten unterschiedliche Feedback-Stimuli erzeugt werden, wobei wenigstens einer der Feedback-Stimuli ein Feedback-Stimulus ist, der den Belastungsbereich für den Patienten angibt. Dem Patienten ist es somit möglich ein Feedback über den Belastungsbereich, in dem sich die Messgröße des Messsignals der Sensoren befindet, zu bekommen.

Vorteilhaft wird mittels eines externen Bediengeräts ein Faktor definiert, durch den die Grenzwerte angepasst werden. Dem Patienten ist es dadurch möglich, während des Tragens des Handschuhs, die Belastungsgrenzen gemäß den individuellen Wünschen entsprechend zu justieren. Situationsbedingte, bevorzugte Feedback-Stimuli können auf diese Weise in den Fokus des Patienten gebracht werden oder derart reduziert werden, sodass der Patient, abhängig von der Situation in der er sich befindet, den Feedback-Stimulus als nicht störend empfindet.

In einer weiteren vorteilhaften Ausgestaltung werden Grenzwerte in Abhängigkeit von der räumlichen Lage der Prothese eingestellt, wobei vorzugsweise die Lage der Prothese mittels eines Neigungssensors erkannt wird. Die Ursprungsachse wird durch den Nutzer definiert. Davon ausgehend wird die jeweilige Winkeländerung gemessen. Eine situationsbedingte Einstellung der Grenzwerte wird dadurch möglich, sodass sich beispielhaft Grenzwerte in einer horizontalen Ebene der Prothese, um z.B. eine Hand zu schütteln oder ein Glas anzuheben, gegenüber Grenzwerten einer vertikalen Lage der Prothese, um z.B. eine Box anzuheben oder ein anderes Gewicht, differenzieren kann.

Nachfolgend werden das erfindungsgemäße Rückmeldungssystem und das entsprechende Verfahren anhand eines Ausführungsbeispiels, das in der Zeichnung schematisch dargestellt ist, näher beschrieben. Hierbei zeigt:
- Fig. 1a: eine mögliche Ausgestaltung eines Rückmeldungssystems;
- Fig. 1b: eine weitere mögliche Ausgestaltung eines Rückmeldungssystems;
- Fig. 2: ein Messsignal über die Zeit und
- Fig. 3: ein Flussdiagramm der Verarbeitungsabfolge von der Erfassung eines Messsignals bis zur Ausgabe mindestens eines Feedback-Stimulus an einen Patienten.

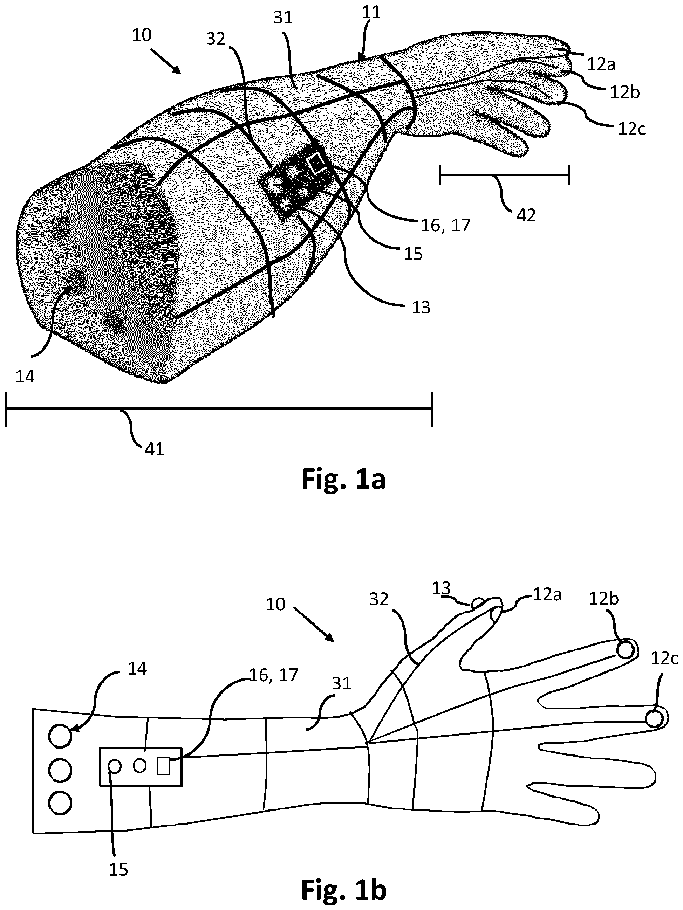

Das in Fig. 1a dargestellte Rückmeldungssystem 10 wird auf einer nicht dargestellten Unterarmprothese eines Patienten 100 getragen. Der Patient 100, im Sinne der vorliegenden Erfindung, ist eine Person, bei der ein Körperglied durch eine Prothese ersetzt ist.

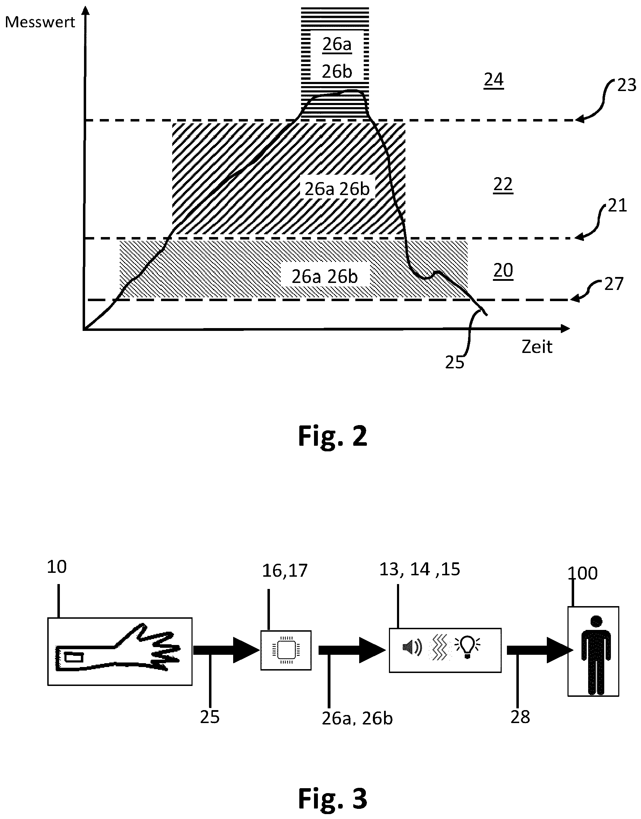

Das Rückmeldungssystem 10 umfasst einen Handschuh 11, der im Bereich der Fingerspitzen Sensoren 12a, 12b, 12c zum Erfassen eines Messsignals 25 aufweist. Wie in Fig. 3 dargestellt ist, übergeben die Sensoren 12a, 12b, 12c das erfasste Messsignal 25 an die verbundene Verarbeitungseinheit 16, welche zusätzlich eine Speichereinheit 17 aufweist. Die Speichereinheit 17 kann das Messsignal 25 und zusätzliche Grenzwerte 21, 23, die durch den Patienten definiert werden können, temporär speichern. Nachdem die Verarbeitungseinheit 16 das Messsignal 25 verarbeitet hat, übermittelt die Verarbeitungseinheit 16 ein Feedback-Signal 26a, 26b an die Rückmeldungseinheiten 13, 14, 15. Die Rückmeldungseinheiten 13, 14, 15 emittieren anschließend einen Feedback-Stimulus 28, der von einem Sinnesorgan des Patienten 100 wahrgenommen werden kann.

Der Handschuh 11 dient als Trägerelement, um die Sensoren 12a, 12b, 12c, die Verarbeitungseinheit 16, die Speichereinheit 17 und die Rückmeldungseinheiten 13, 14, 15 zu tragen, zu positionieren und vor Umwelteinflüssen zu schützen.

Für den Schutz vor Umwelteinflüssen weist der Handschuh 11 ein erstes Material 31 auf, welches sich durch eine geschlossene Oberfläche auszeichnet. Damit weiterhin eine hohe Beweglichkeit und Funktionsfähigkeit der Prothese gewährleistet werden kann, besteht insbesondere das erste Material 31 aus einem weichen Material mit einer hohen Elastizität, beispielsweise PVC oder Silikon. Aufgrund der geschlossenen Oberfläche kann der Handschuh 11 Umwelteinflüsse und Verunreinigungen, wie Feuchtigkeit oder Staub, von der Prothese und insbesondere den Sensoren 12a, 12b, 12c fernhalten.

Zum Positionieren der Einheiten auf dem Handschuh ist ein zweites Material 32 vorgesehen, wie zum Beispiel ein Textil oder eine Faser. Das zweite Material 32 weist ebenfalls eine Elastizität auf, die geringer ist als die Elastizität des ersten Materials 31.

Des Weiteren ist der Handschuh 11 in einen proximalen Bereich 41 und einen distalen Bereich 42 aufgeteilt. Im distalen Bereich 42 sind in vorteilhafter Weise die Sensoren 12a, 12b, 12c vorgesehen, wohingegen im proximalen Bereich 41 die Einheiten zur Verarbeitung des Messsignals 25, nämlich die Verarbeitungseinheit 16, die Speichereinheit 17 und die Rückmeldungseinheiten 13, 14, 15, vorgesehen sind.

Der Schwerpunkt des Rückmeldungssystems 10 liegt im proximalen Bereich 41, da die Einheiten zur Verarbeitung des Messsignals 25 ebenfalls im proximalen Bereich 41 angeordnet sind. Der Abstand zwischen dem Schwerpunkt des Rückmeldungssystems 10 und einem Ellenbogen des Patienten 100 verkürzt sich, sodass bei einer Rotation des Rückmeldungssystems 10 um den Ellenbogen ein geringeres Trägheitsmoment anliegt.

Im distalen Bereich 42 des Handschuhs 11 sind die Sensoren 12a, 12b, 12c angebracht, sodass beispielsweise eine Greifkraft gemessen werden kann. Die Sensoren 12a, 12b, 12c können hierzu ausgebildet sein, Druck, Kapazität, Temperatur, Spannung oder eine sonstige Größe zur Erzeugung eines Messsignals 25 zu erfassen.

Wie zuvor beschrieben, befinden sich die Sensoren 12a, 12b, 12c im Bereich einer Fingerspitze. In Fig. 1a und Fig. 1b befindet sich der Sensor 12a an einem Daumen, der Sensor 12b an einem Zeigefinger und der Sensor 12c an einem Mittelfinger. Ferner ist es auch denkbar, an allen Fingerspitzen einer Hand entsprechende Sensoren 12a, 12b, 12c anzubringen. Ebenfalls ist es denkbar, Sensoren 12a, 12b, 12c in das erste Material 31 einzuarbeiten, sodass diese über die gesamte Handfläche und auf dem Handrücken verteilt angeordnet sind. Die Sensoren 12a, 12b, 12c können ferner auf der außenliegenden Seite des Handschuhs 11 oder auf der innenliegenden Seite des Handschuhs 11 angebracht sein.

Die Verarbeitungseinheit 16 empfängt das erfasste Messsignal 25 der Sensoren 12a, 12b, 12c, verarbeitet dieses und gibt anschließend ein Feedback-Signal 26a, 26b an die Rückmeldungseinheiten 13, 14, 15 ab. Hierzu kann die Verarbeitungseinheit 16 ausgebildet sein, das Feedback-Signal 26a, 26b an die Rückmeldungseinheiten 13, 14, 15 über ein kabelloses Datenübertragungsmedium zu übermitteln. Beispielhaft kann somit eine Rückmeldungseinheit 13, 14, 15 auch in der Hosentasche des Patienten getragen werden, während die andere Rückmeldungseinheit 13, 14, 15 in einer Hand gehalten wird.

Zur Übermittlung des Feedback-Stimulus 28 umfassen die Rückmeldungseinheiten 13, 14, 15 beispielhaft eine oder mehrere LED 13, einen Druckaktuator, welcher mit dem Körper des Patienten derart verbunden ist, sodass ein wahrnehmbarer Druck auf den Patienten ausgeübt werden kann, einen Lautsprecher 15, welcher einen für den Patienten wahrnehmbaren akustischen Ton oder eine Melodie abgeben kann oder einen Vibrationsmotor 14, der ein Frequenzsignal von beispielhaft 200 Hz an den Patienten über die Prothese an den Patienten abgeben kann. Die Rückmeldungseinheit 13, 14, 15 könnte auch ein Peltier-Element zur Anzeige einer Temperaturänderung aufweisen.

In Fig. 1b ist beispielhaft ein weiteres mögliches Ausführungsbeispiel gezeigt, bei der die LED 13 an der Rückseite des Daumens positioniert ist. In dieser und in den nachfolgenden Figuren werden die bereits oben verwendeten Begriffe für gleiche oder funktionsgleiche Teile verwendet.

In Fig. 2 ist beispielhaft ein Verlauf des Messsignals 25 über die Zeit dargestellt. In dieser Darstellung ist der erste Grenzwert 21, der zweite Grenzwert 23, der größer als der erste Grenzwert 21 ist, und ein Schwellenwert 27 eingezeichnet.

Unterhalb des ersten Grenzwerts 21 liegt der erste Belastungsbereich 20. Zwischen dem ersten Grenzwert 21 und dem zweiten Grenzwert 23 liegt der zweite Belastungsbereich 22. Oberhalb des zweiten Grenzwertes 23 liegt der dritte Belastungsbereich 24.

Der Schwellenwert 27 ist kleiner als der erste Grenzwert 21 und befindet sich unter dem ersten Belastungsbereich 20. Der Schwellenwert 27 bestimmt die unterste Grenze, ab welcher ein Feedback-Signal 26a, 26b an die Rückmeldungseinheiten 13,14, 15 übermittelt wird.

Die Ist-Größe des Messsignals 25 bestimmt das Feedback-Signal 26a, 26b abhängig von dem Belastungsbereich 20, 22, 24 in dem sich die Ist-Größe des Messsignals 25 befindet. Folglich wird demnach ein unterschiedliches Feedback-Signal 26a, 26b erzeugt, welches wiederum an die Rückmeldungseinheiten 13, 14, 15 übermittelt wird, wobei die Rückmeldungseinheiten 13, 14, 15 einen Feedback-Stimulus 28 an den Patienten emittieren.

Während sich beispielsweise das Messsignals 25 im ersten Belastungsbereich 20 befindet, wird ein kontinuierliches Feedback-Signal 26a, 26b an die Rückmeldungseinheiten 13,14, 15 ausgegeben. Sobald das Messsignal 25 den ersten Grenzwert 21 überschreitet, wird ein weiteres kontinuierliches Feedback-Signal 26a, 26b an die Rückmeldungseinheiten 13,14, 15 weitergegeben. Das Feedback-Signal 26a, 26b, welches den ersten Belastungsbereich 20 anzeigt, unterscheidet sich zu dem Feedback-Signal 26a, 26b, welches den zweiten Belastungsbereich 22 anzeigt, wodurch ein unstetiges Feedback-Signal 26a, 26b erzeugt wird.

Ein kontinuierliches Feedback-Signal 26a, 26b ist in dem Sinne zu verstehen, dass ein gleichbleibendes Feedback-Signal 26a, 26b an die Rückmeldungseinheit 13, 14, 15 gesendet wird, sodass die Rückmeldungseinheiten 13, 14, 15 jeweils einen Feedback-Stimulus 28 erzeugen, der in der Abfolge der Frequenz, Lautstärke, Lichtintensität oder einer anderen charakterisierenden Eigenschaft gleichbleibend ist. Ein solcher Feedback-Stimulus 28 kann auch Teilbereiche enthalten, in denen kein wahrnehmbarer Feedback-Stimulus 28 übermittelt wird.

Das unstetige Feedback-Signal 26a, 26b ist als ein Feedback-Signal 26a, 26b zu verstehen, welches sich sprunghaft ändert. Bei dem vorliegenden Rückmeldungssystem 10 tritt dieser Effekt auf, sobald der Messwert 25 in einen anderen Messbereich 20, 22, 24 gelangt oder ein Grenzwert 21, 23 überschritten wird.

Aufgrund der sprunghaften Änderung wird es dem Patienten 100 deutlich gemacht, dass der erste Grenzwert 21 überschritten wurde. Analog wird ein Überschreiten des zweiten Grenzwerts 23 dem Patienten 100 kenntlich gemacht. Dem Patienten 100 steht somit ein kontinuierliches Feedback-Signal 26a, 26b zur Verfügung, da das Messsignal 25 sich stets in einem Belastungsbereich 20, 22, 24 befindet, es sei denn, dass ein Schwellenwert 27 definiert ist, der den ersten Belastungsbereich 20 nach unten begrenzt.

Deutliche Änderungen im Feedback-Signal 26a, 26b eignen sich besonders für den taktilen Feedback-Stimulus 28, aber auch für einen visuellen Feedback-Stimulus 28, da dieser mit nur einer geringen Konzentration wahrgenommen werden kann. In Folge dessen ergibt sich ein intuitives System, das die Akzeptanz und die Benutzerfreundlichkeit für den Patienten 100 deutlich erhöht.

In einer weiteren vorteilhaften Ausgestaltung kann der zweite Belastungsbereich 22 durch zusätzliche Grenzwerte feiner unterteilt werden. Der jeweilige Belastungsbereich sendet ebenfalls, wie in dem vorhergehenden Beispiel beschrieben, unterschiedliche Feedback-Signale 26a, 26b an die Rückmeldungseinheiten 13, 14, 15, sodass mittels den unterschiedlichen Feedback-Stimuli 28 dem Patienten 100 kenntlich gemacht wird, in welchem Belastungsbereich sich das Messsignal 25 befindet.

Mittels eines externen mobilen Telefons kann der Grenzwert 21, 23 individuell eingestellt werden. Hierzu kann das externe mobile Telefon mit der Verarbeitungseinheit 16 gekoppelt werden. Zur Justierung der Grenzwerte 21, 23 ist ein Faktor vorgesehen, sodass diese relativ zueinander eingestellt werden können. Des Weiteren können die Grenzwerte 21, 23 aber auch individuell festgelegt werden.

In einer weiteren nicht dargestellten vorteilhaften Ausgestaltung wird mittels eines Neigungssensors die Lage der Prothese erfasst. Der Neigungssensor kann in der Verarbeitungseinheit 16 enthalten sein. Folglich können relativ zu der Lage der Prothese unterschiedliche Grenzwerte 21, 23 definiert werden, sodass beispielhaft bei einer waagerechten Haltung andere Grenzwerte 21, 23 als bei einer senkrechten Haltung der Prothese zur Bestimmung der Belastungsbereiche 20, 22, 24 bestimmt werden.

Mithilfe des Rückmeldungssystems 10 ist es dem Patienten möglich, in seinem Alltag, Feedback-Stimuli 28 zu erleben, die eine einfache Kontrolle von insbesondere der Greifkraft der Prothese ermöglichen. Vor allem ist es mit dem Rückmeldungssystem 10 möglich, dass der Patient 100 auf intuitive Weise die benötigte Greifkraft anlernen und abstimmen kann. Eine intuitive Handhabung der Prothese wird somit deutlich verbessert im Vergleich zu den bisherigen auf dem Markt befindlichen Rückmeldungssystemen für Prothesen

Entgegen bestehender sensorischer Feedback-Systeme, ermöglicht das erfindungsgemäße System die Verarbeitung der Messsignale 25 in verschiedenen Situationen, entweder durch automatische Anpassung des Systems an die gegenwärtige Situation, durch eine Änderung des Modus durch den Benutzer oder mittels selektiver Fokussierung des Patienten 100 auf den auswählten Feedback-Stimulus 28. Die Flexibilität der zur Verfügung stehenden Feedback-Stimuli 28 beeinflusst die Akzeptanz des Systems bei dem Patienten 100 positiv und somit auch die Benutzerfreundlichkeit. Je mehr das System benutzt wird und je mehr Information das Gehirn von dem System erhält, umso mehr kann sich das System in der normalen Funktionsweise des Gehirns einer Person integrieren. Die Basis hierfür liegt in der "Plastizität des Gehirns", z. B. in der Fähigkeit des Gehirns, sich anzupassen oder auch zu lernen. Am Ende führt diese Integration zu einer Veränderung in der Wahrnehmung der Prothese aus der Sicht des Patienten 100, so weit, dass sie als ein Teil des Körpers wahrgenommen werden kann. Somit kann das Risiko der Abstoßung von teuren Prothesen verhindert werden. Eine solche Verbesserung in der Wahrnehmung der Prothese ist auch für eine verbesserte Selbstwahrnehmung des Patienten 100 und einer leichteren sozialen Reintegration notwendig.

### Bezugszeichenliste

- 10: Rückmeldungssystem
- 11: Handschuh
- 12a: Sensor
- 12b: Sensor
- 12c: Sensor
- 13: visuelle Rückmeldungseinheit
- 14: taktile Rückmeldungseinheit
- 15: akustische Rückmeldungseinheit
- 16: Verarbeitungseinheit
- 17: Speichereinheit

- 20: erster Belastungsbereich
- 21: erster Grenzwert
- 22: zweiter Belastungsbereich
- 23: zweiter Grenzwert
- 24: dritter Belastungsbereich
- 25: Messsignal
- 26a: erstes Feedback-Signal
- 26b: zweites Feedback-Signal
- 27: Schwellenwert
- 28: Feedback-Stimulus

- 31: erstes Material
- 32: zweites Material

- 41: proximaler Bereich
- 42: distaler Bereich

- 100: Patient

## Patentansprüche

1. Rückmeldungssystem (10) für eine Prothese, insbesondere für eine Armprothese, eines Patienten (100), umfassend:
einen Handschuh (11), der die Prothese des Patienten (100) zumindest teilweise umschließt;
mindestens einen Sensor (12a, 12b, 12c), der an dem Handschuh (11) angeordnet ist und ein Messsignal (25) erzeugt;
wenigstens eine Rückmeldungseinheit (13, 14, 15), durch die ein Sinnesorgan des Patienten (100) mittels eines Feedback-Stimulus (28) stimuliert wird, und
eine Verarbeitungseinheit (16), die das Messsignal (25) in ein Feedback-Signal (26a, 26b) für die Rückmeldungseinheit (13, 14, 15) umwandelt;
**gekennzeichnet durch** wenigstens zwei einander ergänzende Rückmeldungseinheiten (13, 14, 15), die kontinuierlich jeweils wenigstens einen Feedback-Stimulus (28) erzeugen, durch den mindestens ein Sinnesorgan des Patienten (100) mittels eines akustischen, vibrierenden, optischen und/oder elektrischen Stimulus stimuliert wird, und
eine Speichereinheit (17), die das Messsignal (25) und vorgegebene Grenzwerte (21, 23), die wenigstens drei Belastungsbereiche (20, 22, 24) voneinander abgrenzen, speichert;
wobei die Grenzwerte (21, 23) mindestens einen ersten Grenzwert (21) und einen zweiten Grenzwert (23), der größer als der erste Grenzwert (21) ist, umfassen;
wobei der erste Belastungsbereich (20) unterhalb des ersten Grenzwerts (21), der zweite Belastungsbereich (22) zwischen dem ersten Grenzwert (21) und dem zweiten Grenzwert (23) und der dritte Belastungsbereich (24) oberhalb des zweiten Grenzwerts (23) liegt; und
wobei die Rückmeldungseinheiten (13, 14, 15) derart ausgestaltet sind, dass aus dem Feedback-Signal (26a, 26b) wenigstens ein Feedback-Stimulus (28) erzeugt wird, der dem Patienten den Belastungsbereich (20, 22, 24) angibt.

2. Rückmeldungssystem (10) nach Anspruch 1, **dadurch gekennzeichnet, dass** das Feedback-Signal (26a, 26b) unstetig ist.

3. Rückmeldungssystem (10) nach Anspruch 1 oder 2, **gekennzeichnet durch** einen Schwellenwert (27), der den ersten Belastungsbereich (20) begrenzt.

4. Rückmeldungssystem (10) nach einem der Ansprüche 1 bis 3, **dadurch gekennzeichnet, dass** der Handschuh (11) ein erstes Material (31), insbesondere Silikon oder Polyvinylchlorid, das einen ersten Elastizitätsmodul hat, und wenigstens ein zweites Material (32), insbesondere Textil oder Faser, das einen zweiten Elastizitätsmodul hat, der größer ist als der erste Elastizitätsmodul, umfasst;
wobei vorzugsweise das erste Material (31) und das zweite Material (32) übereinander und/oder nebeneinander angeordnet sind.

5. Rückmeldungssystem (10) nach einem der Ansprüche 1 bis 4, **dadurch gekennzeichnet, dass** der Sensor (12a, 12b, 12c) ausgestaltet ist, Druck, Kapazität, Temperatur, Spannung oder eine sonstige Größe, die geeignet ist, einen wahrnehmbaren Kontakt und/oder Nahezukontakt zu erfassen, zu messen.

6. Rückmeldungssystem (10) nach einem der Ansprüche 1 bis 5, **dadurch gekennzeichnet, dass** der Sensor (12a, 12b, 12c) im Bereich des dritten Fingergliedes der Finger der Prothese, insbesondere im Bereich des dritten Fingergliedes des Daumens, angeordnet ist.

7. Rückmeldungssystem nach einem der Ansprüche 1 bis 6, **dadurch gekennzeichnet, dass** der Sensor (12a, 12b, 12c) in den Handschuh (11) integriert ist.

8. Rückmeldungssystem nach einem der Ansprüche 1 bis 7, **dadurch gekennzeichnet, dass** der Handschuh (11) eine obere Seite, die den Handrücken abdeckt, und eine untere Seite, welche die Handfläche abdeckt, hat;
wobei vorzugsweise der Sensor (12a, 12b, 12c) sich an der unteren Seite des Handschuhs (11) befindet.

9. Rückmeldungssystem (10) nach einem der Ansprüche 1 bis 8, **gekennzeichnet durch** eine Vielzahl an Sensoren (12a, 12b, 12c), die vorzugsweise als Array angeordnet sind;
wobei ferner vorzugsweise die Sensoren (12a, 12b, 12c) auf dem Handschuh (11) verteilt angeordnet sind.

10. Rückmeldungssystem (10) nach einem der Ansprüche 1 bis 9, **dadurch gekennzeichnet, dass** der Handschuh (11) einen die Finger umfassenden distalen Bereich (42) und einen proximalen Bereich (41) aufweist;
wobei vorzugsweise die Verarbeitungseinheit (16) im proximalen Bereich (41) des Handschuhs (11) angeordnet ist.

11. Rückmeldungssystem (10) nach einem der Ansprüche 1 bis 10, **dadurch gekennzeichnet, dass** die wenigstens zwei Rückmeldungseinheiten (13, 14, 15) und die Verarbeitungseinheit (16) mittels eines kabellosen Datenübertragungsmediums miteinander verbunden sind.

12. Rückmeldungssystem (10) nach einem der Ansprüche 1 bis 11, **dadurch gekennzeichnet, dass** die wenigstens zwei Rückmeldungseinheiten (13, 14, 15) als Leuchtelement, vorzugsweise LED (13), als Vibrationsmotor (14), als Druckaktuator und/oder als Lautsprecher (15) ausgebildet sind.

13. Verfahren zum Erzeugen eines Stimulus für einen Patienten mittels eines Rückmeldungssystems (10) nach einem der Ansprüche 1 bis 12, **dadurch gekennzeichnet, dass** aus dem wenigstens einen Feedback-Signal (26a, 26b) durch die wenigstens zwei Rückmeldungseinheiten (13, 14, 15) unterschiedliche Feedback-Stimuli (28) erzeugt werden, wobei wenigstens einer der Feedback-Stimuli (28) ein Feedback-Stimulus (28) ist, der dem Patienten den Belastungsbereich (20, 22, 24) angibt.

14. Verfahren nach Anspruch 13, **dadurch gekennzeichnet, dass** mittels der Verarbeitungseinheit (16) oder mittels eines externen Bediengeräts ein Faktor definiert wird, durch den die Grenzwerte (21, 23) angepasst werden.

15. Verfahren nach Anspruch 13 oder 14, **dadurch gekennzeichnet, dass** die Grenzwerte (21, 23) in Abhängigkeit von der räumlichen Lage der Prothese eingestellt werden;
wobei vorzugsweise die Lage der Prothese mittels eines Neigungssensors erkannt wird.
